# EUROPEAN PATENT SPECIFICATION

(11) **EP 2 149 378 B1**
(45) Date of publication and mention of the grant of the patent: **06.08.2014**
(21) Application number: 08171353.9
(22) Date of filing: 11.12.2008
(51) Int. Cl.: A61K 36/324, A61K 36/81, A61K 36/77, A61P 19/00

(54) **Topical formulations for the symptomatic treatment of musculoskeletal disorders**
Topische Formulierungen für die symptomatische Behandlung von Skelettmuskelkrankheiten
Formulations topiques pour le traitement symptomatiques de troubles musculo-squelettiques

(30) Priority: 29.07.2008 IT MI20081404
(43) Date of publication of application: 03.02.2010
(73) Proprietor: Velleja Research SRL, 29010 Pontenure (PC) (IT)
(72) Inventor: Di Pierro, Francesco, 29010, PONTENURE (PC) (IT)
(74) Representative: Minoja, Fabrizio

(56) References cited:
- LONG L ET AL: "Herbal medicines for the treatment of osteoarthritis: A systematic review" RHEUMATOLOGY, OXFORD UNIVERSITY PRESS, LONDON, GB, vol. 40, no. 7, 1 July 2001 (2001-07-01), pages 779-793, XP009106094 ISSN: 1462-0324
- MELETIS C D ET AL: "RHEUMATOID ARTHRITIS ETIOLOGY AND NATUROPATHIC TREATMENTS" ALTERNATIVE AND COMPLEMENTARY THERAPIES, MARY ANN LIEBERT, LARCHMONT, NY, US, vol. 7, no. 6, 1 December 2001 (2001-12-01), pages 347-354, XP001126222 ISSN: 1076-2809
- MENVIELLE-BOURG F J: "Plants and ageing, recent data. PLANTES ET VIEILLISSEMENT, DONNEES ACTUELLES" PHYTOTHÉRAPIE, SPRINGER-VERLAG, PARIS, FR, vol. 3, no. 2, 1 January 2005 (2005-01-01), pages 57-71, XP002407507 ISSN: 1624-8597
- GUILLEMIN R ET AL: "[Trial use of aescine by drug dielectrolysis--apropos of 80 cases]" 1977, ELECTRODIAGNOSTIC-THÉRAPIE 1977, VOL. 14, NR. 2, PAGE(S) 51 - 54 , XP009115978 ISSN: 0424-8120 * the whole document *
- CLAYTON J J: "Nutraceuticals in the management of osteoarthritis" ORTHOPEDICS, THOROFARE, NJ, US, vol. 30, no. 8, 1 August 2007 (2007-08-01), pages 624-629, XP009115930 ISSN: 0147-7447
- STYCZYNSKI T: "Degenerative stenosis of lumbar spinal canal with neurogenic claudication. Pathomechanism of development of clinical symptoms and therapeutical implications" REUMATOLOGIA (WARSAW),, vol. 42, no. 1, 1 January 2004 (2004-01-01), pages 59-63, XP002525357

## Description

The present invention relates to topical compositions or extracts containing capsaicin, boswellic acids or *Boswellia serrata* extracts, and escin or *Aesculus hippocastanum* extracts.

### Prior art

Musculoskeletal disorders are always associated with joint damage and pain. They are consequently defined as painful musculoskeletal disorders. They include a large number of disorders, ranging from peripheral joint diseases (such as rheumatic disease, rheumatoid arthritis, connective tissue disease, osteoarthritis, gout, sarcoidosis and infectious arthritis) to myalgia (such as polymyalgia rheumatica, ankylosing spondylitis and psychogenic rheumatism), or joint, neurological, degenerative, autoimmune, vascular and traumatic disorders which can involve the joints, limbs, ankles and lumbosacral region, causing pain and functional impairment.

The most common degenerative inflammatory disease of the musculoskeletal apparatus is osteoarthritis (OA), also known as degenerative joint disease.

This disease affects women more than men up to the age of 50 years. After that age the percentages are equivalent, but complications are more frequent in men. Alterations may also occur in healthy young people in their twenties (rare), whereas more than 60% of the population aged over 60 present joint alterations of varying extents. Over 20 million people in the USA suffer from this disease, and at least 200,000 hip and knee replacement operations are caused by osteoarthritis every year.

The etiology of the disease has not yet been identified, but it has strong links with genetic factors, obesity and mechanical stress on the joint. In particular, trauma affecting the cartilage surface of the joint or trauma of the subchondral bone can create cartilage microfractures and give rise to the disease. Occupational fatigue or microtraumas caused by sport can also operate with the same mechanism. An altered immune response can also damage the cartilage surface, causing the chondrocytes to release proteolytic and collagenolytic enzymes which degrade collagen and proteoglycans and cause subsequent synthesis of bone repair tissue with the formation of osteophytes. The deposit of calcium pyrophosphate dihydrate (CPPD) microcrystals triggers inflammatory symptoms, with the release of proteolytic enzymes that cause cartilage damage. A number of endocrine disorders can cause the deposit of CPPD crystals: hyperparathyroidism, Wilson's disease, haemochromatosis and hypothyroidism. The anatomopathological damage is represented by cracking with erosion of the cartilage, and formation of areas wholly devoid of cartilage. The proliferative response of the bone tissue is once again sclerosis, thickening of the subchondral bone, and production of osteophytes.

In the case of OA, the joints most affected are the small joints of the hands (first carpometacarpal joints, proximal and distal interphalangeal joints) and feet (metatarsophalangeal joints), and the hip, knee and spine joints. The main symptoms are progressive pain, which is initially mild, but later becomes constant and disabling, crepitation of the affected joints, joint deformity due to increased bone tissue in the synovial fluid and synovitis, limited joint movements with spasms, and joint instability. Valgus deformity of the knee may also be present. The disease has a chronic-degenerative trend. The painkilling drugs most commonly used are paracetamol, acetylsalicylic acid, NSAIDs, indometacin, methotrexate and cyclosporin A, which present serious side effects such as liver disease, kidney disease, gastroenteric ulcers, and immunosuppression. The same drugs are used for nearly all painful rheumatic syndromes, especially in rheumatoid arthritis, where the anatomopathological damage leads to clinical symptoms substantially similar to those of osteoarthritis.

The transcutaneous approach, using topical preparations or medical devices that release the drug, involves a lower incidence of side effects and also allows a lower dose of the drugs to be used than with the oral route.

Particularly favourable results have been reported with capsaicin-based preparations.

Capsaicin is present, at different concentrations, in plants of the genus Capsicum. Capsaicin and capsaicinoids are very stable alkaloids: they remain unchanged for a long time, even after cooking and freezing. Like all capsaicinoids, capsaicin is an irritant, and produces a stinging sensation in the mucous membranes, where it passes into solution and stimulates the VR1 receptors (vanilloid receptor type 1), which in turn activate the protein VRL-1 (vanilloid receptor-like 1), which under "normal" conditions is activated at temperatures of between 43 and 52°C.

Although capsaicin is rather toxic by oral administration, if administered topically at a very low dose it effectively reduces pain symptoms due to its local analgesic activity, but has no effect on the inflammatory component of the disorder.

### Description of the invention

It has now surprisingly been found that the topical activity of capsaicin in reducing the symptoms of osteoarthritis and similar chronic osteoarticular symptoms can be improved by combining it with escin and boswellic acids or extracts containing them.

The invention therefore provides topical compositions containing:
a) capsaicin or extracts containing it,
b) boswellic acids or *Boswellia serrata* resin extracts containing them (preferably a 60% *Boswellia* extract in boswellic acids),
c) escin in free form or complexed with beta-sitosterol and/or with phospholipids or *Aesculus hippocastanumseed* extracts.

*Boswellia serrata* Roxb., also known as Indian frankincense, is a tree belonging to the *Burseraceae* family which is native to the forests of some parts of India, North Africa and the Orient. The constituent most widely used for medicinal purposes is a yellow-brown rubbery oleoresin also called gum resin or "guggal", obtained by incision of the bark or extraction from the leaves. *Boswellia serrata* gum resin and alcoholic extracts thereof are used as anti-inflammatory remedies in the treatment of disorders such as ulcerating colitis, bronchial asthma and pulmonary emphysema. *Boswellia* contains numerous terpenoid substances, polysaccharides, uronic acids, β-sitosterol and phlobaphenes, which are used to treat various disorders. The terpenoid fraction consists of pentacyclic triterpenic acids called boswellic acids, which are the main active constituents. The acids are classified as α-, β- and γ-boswellic acids. The β form is the predominant one. The extracts available on the market are standardised in boswellic acids with percentages of between 37 and 65%. The dried extract content, titrated in acetyl-11-keto-β-boswellic acid (AKBA), the most active constituent, is 30%. Boswellic acids, the active constituents of *Boswellia serrata,* perform a strong anti-inflammatory action and act similarly to a non-steroidal anti-inflammatory drug. *In vivo* studies conducted on rats have demonstrated their ability to reduce oedema caused by local inoculation of carrageenan. Experiments conducted *in vitro* demonstrate that *Boswellia* extracts inhibit, to a dose-dependent extent, synthesis of products of the enzyme 5-lipoxygenase, such as 5-hydroxyeicosatetraenoic acid (5-HETE) and leukotriene B4 (LTB4), which are responsible for bronchoconstriction, stimulating chemotaxis and increasing vascular permeability, with consequent oedema formation. In particular, AKBA is a powerful inhibitor of leukotriene synthesis, acting on 5-lipoxygenase with a direct, non-redox and non-competitive mechanism: it is the only compound currently known which acts as allosteric regulator of the enzyme. *Boswellia* derivatives are consequently considered to be specific inhibitors of 5-lipoxygenase. It has also been observed that boswellic acids perform an inhibitory action towards human leucocyte elastase (HLE), an enzyme that stimulates mucus production in the respiratory apparatus. Said enzyme, which seems to play an important part in diseases such as pulmonary cystic fibrosis, chronic bronchitis and acute respiratory distress syndrome, also appears to contribute to the inflammatory symptoms typical of osteoarthritis. Inflammatory processes are characterised by multiple symptoms, such as loss of functionality of the tissue involved, oedema formation, swelling and pain, and are at least partly attributable to the presence of leukotrienes, known inflammation mediators with chemotactic activity, which cause spasms of the smooth muscles and increase vascular permeability. Moreover, the ability of boswellic acids to prevent the migration of polymorphonucleated leucocytes by inhibiting the release or production of some chemotactic factors has recently been demonstrated *in vitro.* Said cells appear to act locally by releasing elastase, a proteolytic enzyme jointly responsible for the destruction of tissues inflamed by chronic degenerative syndromes.

Although oedema has no relevance to the symptoms of the disorder in question, a surprising further synergy of action has been observed in local treatment if preparations with an anti-oedema action are combined with the preparation based on capsaicin and *Boswellia* derivatives. The tissue oedema typical of traumatic events can be treated either with drugs that reactivate the lymphatic circulation, resorb liquids and redirect them to the venous region, or drugs that physically reduce the endothelial slit through which the liquids exit. An example of the second type of drug is escin, a mixture of saponins contained in *Aesculus hippocastanum* seeds; said saponins consist of a mixture of aglycons known as desglucoescin to which one molecule of glucuronic acid and two monosaccharide residues are connected, together with tiglic acid or angelic acid in position 21. Escin possesses excellent anti-oedema properties which make it particularly useful for treating post-traumatic intra-cranial effusions and, in the cosmetic field, for resorbing the tissue oedema typical of cellulitis. The action mechanism of escin is based on its ability to reduce the number and diameter of the endothelial pores responsible for the physiological exchange of liquids between the blood vessels and the surrounding connective tissue. Action at this level, by reducing the exit of interstitial liquids, generates tissue resorption of the oedema. However, after topical administration, in a very small percentage of cases, escin can cause mild irritation due to its ability to sequester cholesterol (which it takes from the cell membranes, causing cytolysis). It is therefore preferable to use escin in the form complexed with beta-sitosterol and/or with distearoyl phosphatidylcholine. Said complexing saturates the cholesterol-binding site and eliminates the risk of irritation without altering the pharmacological properties of pure escin.

The formulation to which this patent relates contains between 0.0001 and 1% by weight of capsaicin, between 0.001 and 5% of 60% *Boswellia* extract in boswellic acids, and between 0.01 and 5% of escin/beta-sitosterol/distearoylphosphatidylcholine complex.

A particularly preferred composition contains 0.075% by weight of capsaicin, 1% by weight of *Boswellia* extract, and 1.5% by weight of escin/beta-sitosterol/distearoyl phosphatidylcholine complex.

The compositions according to the invention may also contain antioxidants such as curcumin, OPC from *Vitis vinifera,* vitamins C and E, lipoic acid and/or preparations which act on the lymphatic system (e.g. melilotus, coumarin, esculoside, fraxetine, etc.) and/or preparations which implement the anti-inflammatory action by acting on endogenous cortisol (18-beta glycyrrhetinic acid, liquorice root extract). The compositions can take the form of creams, gels, hydrocreams, ointments and emulsions. If osteoarticular pain occurs in elderly patients with ulcers and sores close to the inflamed, painful joint, a spray formulation could be used, as its application does not require manual contact with the ulcerated/painful area. As the formulations according to the invention are designed for chronic use, the active constituents may also be applied to devices which effect programmed, long-term release, such as patches, bandages, applicators or simply sterile gauze, which is useful for outdoor use, for example.

The following examples illustrate the invention in greater detail.

### EXAMPLES

### EXAMPLE 1 - Cream (for joints)

| Ingredient | Percentage by weight |
|---|---|
| Capsaicin | 0.075% |
| Boswellic acids | 1.0% |
| Escin/beta-sitosterol/phytosome | 1.5% |

### EXAMPLE 2 - Gel (for hands)

| Ingredient | Percentage by weight |
|---|---|
| Capsaicin | 0.025% |
| Boswellic acids | 0.5% |
| Escin/beta-sitosterol/phytosome | 0.5% |

### EXAMPLE 3 - W/O emulsion (lumbar area and back)

| Ingredient | Percentage by weight |
|---|---|
| Capsaicin | 0.075% |
| Boswellic acids | 1.0% |
| Escin/beta-sitosterol/phytosome | 1.5% |
| OPC from *Vitus vinifera* | 1.0% |
| 18-beta glycyrrhetinic acid phytosome | 1.5% |

### EXAMPLE 4 - Spray

| Ingredient | Percentage by weight |
|---|---|
| Capsaicin | 0.075% |
| Boswellic acids | 0.5% |
| Escin/beta-sitosterol/phytosome | 0.5% |
| Lipoic acid | 0.5% |

### EXAMPLE 5 - Medicated gauze

| Ingredient | Percentage by weight |
|---|---|
| Capsaicin | 0.075% |
| Boswellic acids | 1.5% |
| Escin/beta-sitosterol/phytosome | 1.5% |

### EXAMPLE 6 - Controlled-release patches

| Ingredient | Percentage by weight |
|---|---|
| Capsaicin | 0.075% |
| Boswellic acids | 0.5% |
| Escin/beta-sitosterol/phytosome | 0.5% |
| Curcumin | 0.5% |

## Claims

1. Topical compositions comprising:
a) capsaicin or extracts containing it;
b) boswellic acids or extracts of *Boswellia serrata* resin containing them;
c) escin in the free form or complexed with beta-sitosterol and/or with phospholipids or extracts of *Aesculus hippocastanum* seeds.

2. Compositions as claimed in claim 1, wherein escin is complexed with beta-sitosterol.

3. Compositions as claimed in claim 1, wherein escin is complexed with beta-sitosterol and with distearoyl phosphatidylcholine.

4. Compositions as claimed in any one of claims 1 to 3, wherein the component b) is a *Boswellia* extract with 60% boswellic acids content.

5. Compositions as claimed in any one of claims 1 to 4, wherein the components are present within the following weight percentages:
a) capsaicin: 0.0001 to 1%;
b) *Boswellia* extract with 60% boswellic acids content: 0.001 to 5%;
c) escin/beta-sitosterol/distearoyl phosphatidylcholine complex: 0.01 to 5%.

6. Compositions as claimed in claim 5 wherein the components are present in the following weight percentages:
a) capsaicin 0.075%;
b) *Boswellia* extract: 1%;
c) escin/beta-sitosterol/distearoyl phosphatidylcholine complex: 1,5%.

7. Compositions as claimed in any one of claims 1 to 6, further comprising antioxidizing agents or agents active on the lymphatic system and/or anti-inflammatory agents acting on endogenous cortisol.

8. Compositions as claimed in any one of claims 1 to 7 in the form of creams, gel, water-based creams, ointments, emulsions, spray.

9. Compositions as claimed in any one of claims 1 to 7 in the form of plasters, bandages, applicators and sterile gauzes.

10. The use of:
a) capsaicin or extracts containing it;
b) boswellic acids or extracts of *Boswellia serrata* resin containing them;
c) escin in the free form or complexed form with beta-sitosterol and/or with phospholipids or extracts of *Aesculus hippocastanum* seeds;
for the preparation of topical compositions for the treatment of osteoarthritis

## Patentansprüche

1. Topische Zusammensetzungen umfassend:
a) Capsaicin oder Capsaicin-enthaltende Extrakte;
b) Boswelliasäuren oder Boswelliasäuren- enthaltende Extrakte aus *Bosavellia serrata*-Harz;
c) Escin in der freien Form oder komplexiert mit beta-Sitosterin und/oder mit Phospholipiden oder Extrakten aus *Aesculus hippocastanum*-Samen.

2. Zusammensetzungen wie in Anspruch 1 beansprucht, wobei Escin komplexiert mit beta-Sitosterin ist.

3. Zusammensetzungen wie in Anspruch 1 beansprucht, wobei Escin komplexiert mit beta-Sitosterin und mit Distearoylphosphatidylcholin ist.

4. Zusammensetzungen wie in einem der Ansprüche 1 bis 3 beansprucht, wobei Komponente b) ein *Boswellia*-Extrakt mit 60% Boswelliasäure-Gehalt ist.

5. Zusammensetzungen wie in einem der Ansprüche 1 bis 4 beansprucht, wobei die Komponenten in folgenden Gewichtsprozenten vorliegen:
a) Capsaicin: 0,0001 bis 1%;
b) *Bosavellia*-Extrakt mit 60% Boswelliasäure-Gehalt: 0,001 bis 5%;
c) Escin/beta-Sitosterin/Distearoylphosphatidylcholin-Komplex: 0,01 bis 5%.

6. Zusammensetzungen wie in Anspruch 5 beansprucht, wobei die Komponenten in folgenden Gewichtsprozenten vorliegen:
a) Capsaicin: 0,075%;
b) *Bosavellia*-Extrakt: 1%;
c) Escin/beta-Sitosterin/Distearoylphosphatidylcholin-Komplex: 1,5%.

7. Zusammensetzungen wie in einem der Ansprüche 1 bis 6 beansprucht, ferner umfassend Antioxidationsmittel oder Stoffe, die wirksam auf das lymphatische System sind und/oder antiinflammatorische Stoffe, die auf das endogene Cortisol wirken.

8. Zusammensetzungen wie in einem der Ansprüche 1 bis 7 beansprucht in der Form von Cremes, Gel, Wasser-basierten Cremes, Salben, Emulsionen, Spray.

9. Zusammensetzungen wie in einem der Ansprüche 1 bis 7 beansprucht in der Form von Pflastern, Verbänden, Applikatoren und sterilen Gazen.

10. Die Verwendung von:
a) Capsaicin oder Capsaicin-enthaltenden Extrakten;
b) Boswelliasäuren oder Boswelliasäuren- enthaltenden Extrakten aus *Boswellia serrata-*Harz;
c) Escin in der freien Form oder komplexiert mit beta-Sitosterin und/oder mit Phospholipiden oder Extrakten aus *Aesculus hippocastanum*-Samen;
zur Herstellung von topischen Zusammensetzungen für die Behandlung von Osteoarthritis.

## Revendications

1. Compositions topiques comprenant :
- a) de la capsaïcine ou des extraits la contenant ;
- b) des acides boswelliques ou des extraits de résine de *Boswellia serrata* les contenant ;
- c) de l'escine sous forme libre ou complexée avec du bêta-sitostérol et/ou avec des phospholipides ou des extraits de graines *d'Aesculus hippocastanum.*

2. Compositions tel que revendiqué selon la revendication 1, dans lesquelles l'escine est complexée avec du bêta-sitostérol.

3. Compositions tel que revendiqué selon la revendication 1, dans lesquelles l'escine est complexée avec du bêta-sitostérol et avec de la distéaroyl phosphatidylcholine.

4. Compositions tel que revendiqué selon l'une quelconque des revendications 1 à 3, dans lesquelles le composant b) est un extrait de *Boswellia* avec une teneur en acides boswelliques de 60 %.

5. Compositions tel que revendiqué selon l'une quelconque des revendications 1 à 4, dans lesquelles les composants sont présents selon les pourcentages en poids suivants :
- a) capsaïcine : 0,0001 à 1 % ;
- b) extrait de *Boswellia* avec une teneur en acides boswelliques de 60 % : 0,001 à 5 % ;
- c) complexe d'escine/bêta-sitostérol/distéaroyl phosphatidylcholine : 0,01 à 5%.

6. Compositions tel que revendiqué selon la revendication 5 dans lesquelles les composants sont présents en les pourcentages en poids suivant :
- a) capsaïcine : 0,075 %
- b) extrait de *Boswellia* : 1 % ;
- c) complexe d'escine/bêta-sitostérol/distéaroyl phosphatidylcholine: 1,5%

7. Compositions tel que revendiqué selon l'une quelconque des revendications 1 à 6, comprenant en outre des agents antioxydants ou des agents actifs sur le système lymphatique et /ou des agents anti-inflammatoires agissant sur le cortisol endogène.

8. Compositions tel que revendiqué selon l'une quelconque des revendications 1 à 7 sous la forme de crèmes, de gels, de crèmes à base d'eau, d'onguents, d'émulsions, de pulvérisation.

9. Compositions tel que revendiqué selon l'une quelconque des revendications 1 à 7 sous la forme d'emplâtres, de bandages, d'applicateurs et de gazes stériles.

10. Utilisation :
- a) de capsaïcine ou d'extraits la contenant ;
- b) d'acides boswelliques ou d'extraits de résine de *Boswellia serrata* les contenant ;
- c) d'escine sous forme libre ou sous forme complexée avec du bêta-sitostérol et/ou avec des phospholipides ou des extraits de graines *d'Aesculus hippocastanum ;*
pour la préparation de compositions topiques pour le traitement de l'ostéoarthrite.
